# EUROPEAN PATENT APPLICATION

(11) **EP 3 863 024 A1**
(43) Date of publication of application: **11.08.2021**
(21) Application number: 20156287.3
(22) Date of filing: 10.02.2020
(51) Int. Cl.: G16H 50/50

(54) **BIOSIGNAL MONITORING**

(71) Applicant: Sense4Health Oy, 90910 Kontio (FI)
(72) Inventor: Särkkä, Simo, 90910 Kontio (FI); Arvonen, Miika, 90910 Kontio (FI); Hostettler, Roland, 90910 Kontio (FI)
(74) Representative: Kolster Oy Ab

(57) **Abstract**

The invention relates to an apparatus comprising: a simulating device (202) for obtaining simulated physiological response of a living body to vital functions of the living body; a controlling device (204) for controlling the simulating device. The apparatus further comprises a measuring device (208) for measuring the simulated physiological response to obtain measurement data and adapting device (212) for comparing the measurement data to predetermined data and adapting the measuring and controlling means based on the comparison.

## Description

### FIELD OF THE INVENTION

The present invention relates to simulating biosignal monitoring, for example to simulating of monitoring vital functions or vital signs of a body of a living being.

### BACKGROUND OF THE INVENTION

Monitoring vital functions or vital signs of a body of a living being is as such known in the art. Vital signs are produced by different kinds of vital functions that refer to internal workings of the body causing the vital signs. Vital functions are for example respiration, cardiac functions, and temperature, to name a few. For example for monitoring respiration, there exists various sensing technologies. However, the current monitoring systems are typically based on monitoring of a single parameter, such as the movement of the thorax of the living being monitored. Some multisensor solutions have also been proposed. Examples of respiration monitoring methods include respiration belts and other thorax movement monitoring means such as inertial sensors, optical and thermal camera systems, impedance measurements (such as Electrocardiography, ECG), capacitive sensors (in wearable textiles, for example), radio-frequency measurements (such as radar or signal phase measurements) and audio-based measurement systems, among others.

Cardiac function monitoring is also as such known in the art. The available methods include, for example, ECG-measurements, optical pulse oximeter systems, camera-based measurements, audio-based systems, inertial sensor based systems, and various others. Cardiac monitoring is also typically based on monitoring of single sensor modality at a time although some multimodal measurements systems have been developed as well.

However, there is a need for a more accurate and robust means to monitor respiration and cardiac functions so that data of these functions could be utilised more reliably.

### BRIEF DESCRIPTION OF THE INVENTION

According to an aspect of the present invention, there is provided an apparatus of claim 1.

According to an aspect of the present invention, there is provided a method of claim 10.

One or more examples of implementations are set forth in more detail in the accompanying drawings and the description below. Other features will be apparent from the description and drawings, and from the claims. The embodiments and or examples and features, if any, described in this specification that do not fall under the scope of the independent claims are to be interpreted as examples useful for understanding various embodiments of the invention.

### BRIEF DESCRIPTION OF THE DRAWINGS

In the following the invention will be described in greater detail by means of preferred embodiments with reference to the attached drawings, in which
Figures 1A, 1B, 1C, 1D, 1E and 1F illustrate examples of measuring a vital sign such as breathing pattern of a human body;
Figure 2 illustrates a simplified example of an apparatus of an embodiment;
Figure 3 illustrates a simplified schematic example of an embodiment;
Figures 4A and 4B illustrate an example of an embodiment of a simulator realised with a physical human shape torso or robot;
Figure 5 is a flowchart illustrating an embodiment; and
Figure 6 illustrates an example of a computing device.

### DETAILED DESCRIPTION OF THE INVENTION

As mentioned, vital signs of a body of a living being are produced by different kinds of vital functions that refer to internal workings of the body. The internal workings of the body cause changes in the respiration and cardiac patterns of the body, for example. Detecting and analysing the vital signs may enable the detection of several internal workings of a body. In following, a human body is used as an example, but embodiments may be applied to a body of another kind of living being as well as one skilled in the art is well aware.

For example, different respiration and cardiac patterns may imply respiratory morbidities, level of stress, excitement, mood, sexual arousal, drug abuse, fatigue or awakeness level, seizures related to e.g. asthma, or simply overall health of the person. Both the respiration and cardiac patterns may be indications of these same conditions, but jointly the vital signs tell more about the state of the living entity and its internal vital functions more than either of the modalities alone.

In general, results of monitoring vital signs such as respiration and cardiac functions can, for example, be used for monitoring patients in hospitals as well as home. The measurement results may be used for diagnosing cardiac and other illnesses or for determining the sleeping patterns and quality of living beings. This kind of measurement or monitoring system can also be used to detect seizures such as asthma attacks as well as other types of seizures that affect respiration and cardiac function The main aims of these systems is to monitor the health of the person when something is suspected to be wrong or there is a risk of sudden change in health condition. During sports exercise the systems can monitor breathing and cardiac pattern of the exerciser and warn the exerciser in time to reduce too vigorous physical activity if there is a significant risk of exerciser to get lactic acidosis or asthma attack or other unwanted consequence of the exercise

The proposed solution enables the design and calibration or adjustment of measurement systems which can reliably detect vital signs. In the proposed system a vital sign simulator is used in connection with a measurement system and the measurement system can be taught to interpret sensor readings in a correct manner to obtain reliable indications of vital functions of a human body.

The measurement system calibrated or adjusted utilising the simulator also allows for passive or active monitoring of the fatigue, awakeness, or stress level of a living being even when there is no suspicion of morbidities. This kind of systems are useful, for example, in monitoring of people in their work, while driving a car, analyzing the response to movies or ads, or in identification of suspicious persons in crowds. In addition to human beings, similar analysis can also be used for animals. This kind of system can, for example, automatically generate an alarm, warning, or log entry when the living being is going to fall asleep, when the stress level is high, when they are under the influence of narcotic substances, or when the living being seems tired.

Vital functions such as respiration patterns and cardiac function also tell about the mood and excitement of the monitored living being. In addition to the aforementioned applications, this information can be used to tune the response of a human-computer or animal-computer interface such as chatbot or graphical user interface to respond differently, for example, more softly or more sharply, depending on the mood or excitement. The same technology also allows for monitoring of sexual arousal which can be used both for security purposes as well as for entertainment business and products. The same technology can be used for measurement of rate of coughing and relate the deepness of inspirium before coughing and expirium phase to the strength of coughing.

Passive monitoring of respiration and/or cardiac function also allows for detection of the presence of a living being. In some cases, for example, due to face-mask, rubber face, or other disguise it might be hard to otherwise determine if a particular object is a living being or actually the same person as the face of him/her, but by measuring the presence of respiratory or cardiac pattern and thermal measurement of face, this detection can be done. Facial mask effect on temperature of the face, thus simultaneous measurement of facial and nasal temperature helps to distinguish between real face and a mask or other face cover. Thermal camera could also reveal the real face beneath the mask or cover. It is also possible to track the location or the movement of a person solely on the detected vital signs, for example, by tracking an oscillating temperature spot or spots in thermal camera.

By combining breathing information with the size of pupils it is also possible to detect arousal, sympathetic tonus, and possible exposure to drugs, especially narcotic drugs. In a person exposed to certain psychostimulant narcotics like amphetamine, metamphetamine, ecstasy or cocaine the pupils are enlarged and do not react to the flash light, and their respiratory and heart rhythm is higher than normal. The same psychostimulants cause the body to overheat and causes difficulties to stand in a static position. The individuals which are exposed to psychostimulants are at risk for other persons due to their aggressiveness. The opposite is the case in opiate drug users. They tend to have slower respiratory and heart rhythm and constricted pupils and are in a risk of apnea and death. While all the above mentioned parameters are insufficient on their own, they are useful when combined together to distinguish drug abusers for example for security reasons.

Monitoring increased pupil size, decreased mimics of face, yawning and breathing pattern can help to detect drowsiness of car drivers. This kind of monitoring can also be combined with information on the driving style as well as to tune the amount of control and cautiousness that a driving assistance system takes in an autonomous or semi-autonomous car or other transportation vehicle.

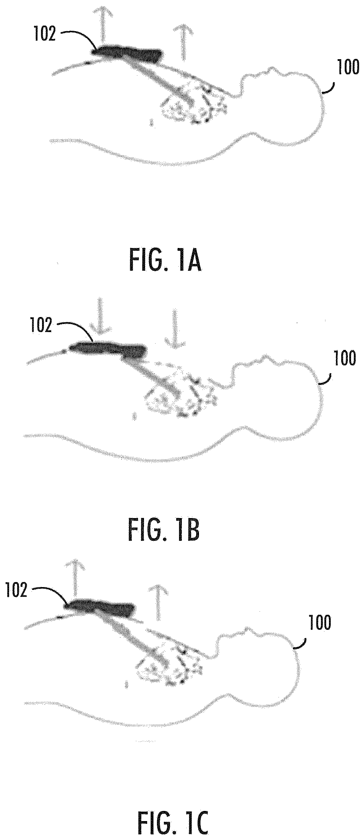

As an example of vital signs and measuring the signs, consider a set of breathing patterns illustrated in Figs. 1A - 1F. Breathing patterns of a human body 100 may be monitored by a sensor 102 placed on the chest of the body. The sensor may be a mobile phone or a sensor device comprising motion sensors and microphone, for example.

Sensor measurement can distinct various breathing patterns by monitoring sensor position and sensor rotational axis, the distance between heart and the sensor device (time lack of the heart motion and detection of it by sensor device accelerometer), heart rhythm variation due to difference of intrathoracic pressure during inspirium and expirium effect on cardiac preload and tidal volume, and consequently to the compensatory variation of heart rhythm (detected by accelerometer).

Fig. 1A illustrates normal inspirium. Normal inspirium causes a bit increased distance between heart and the sensor device compared to expirium phase. The sensor device position is horizontal or rotating slightly and there can be noted a heart rate variation, that is heart rhythm is elevated in inspirium due to elevated intrathoracic pressure to compensate decreases preload and heart tidal blood volume.

Fig. 1B illustrates normal expirium. In normal expirium there is a bit shorter distance between heart and the sensor device, the sensor device position is horizontal or rotating slightly and there can be noted a heart rate variation i.e heart rhythm is decreasing in expirium due to decreased intrathoracic pressure to compensate decreases preload and heart tidal blood volume.

Fig. 1C illustrates alveolar breathing pattern. Here more time is needed for oxygen to diffuse to lung capillaries. Causes may be microathelectasis of the lung, odema in the lungs, or inflammation in the lung tissue (longer way of oxygen to diffuse from alveolar space to capillaries).

Strong inspiratory movement of abdomen causes a bigger distance between heart and the sensor device, the sensor device position is horizontal or rotating slightly. Platau after inspirium (the sensor device is in same position during platau phase) and the heart is constantly elevated. Heart rate variation is lacking due to severe respiratory distress (problems in alveolar oxygenation).

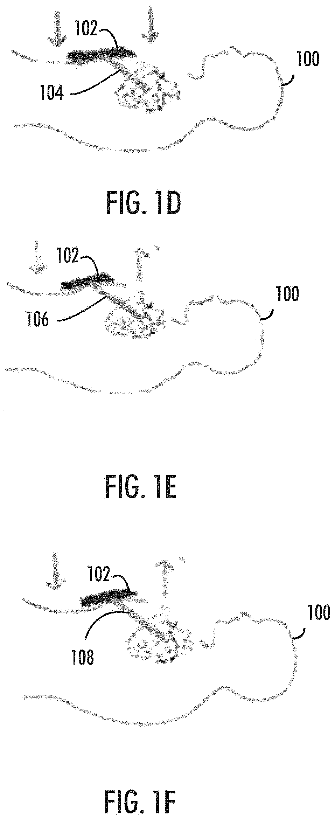

Fig. 1D illustrates obstruction of the bronchus or bronchioles. Deep expirium causes a shorter distance 104 between heart and the sensor device, the sensor device position is horizontal or rotating down towards abdomen from abdominal site, and the heart rhythm is getting a bit slower due to decreased intrathoracic pressure, which elevated the preload in the heart (increases tidal blood volume). Further, inspirium-expirium ratio is over 1:1.

Fig. 1E illustrates paradoxical breathing expirium phase. Here deep abdominal movement simultaneously with thorax going up (paradoxically), causes a very short distance 106 between heart and the sensor device. The sensor device position is rotating clearly down towards abdomen from abdominal site and the heart rhythm is very high. Since a stressful situation due to severe obstruction the heart rhythm remains high (there is a lack of hearth rhythm variation). In this situation the expirium is very long compared to inspirium (inspirium-expirium ratio ≈1:3).

Fig. 1F illustrates paradoxical breathing inspirium phase. Abdominal expansive movement simultaneously with thorax going to opposite direction (paradoxically), causes a long distance 108 between heart and the sensor device. The sensor device position is rotating clearly down towards abdomen from abdominal side, but since there is a stressful situation due to severe obstruction the heart rhythm remains high (there is a lack of hearth rhythm variation). In this situation the inspirium is very short (inspirium-expirium ratio ≈1:3).

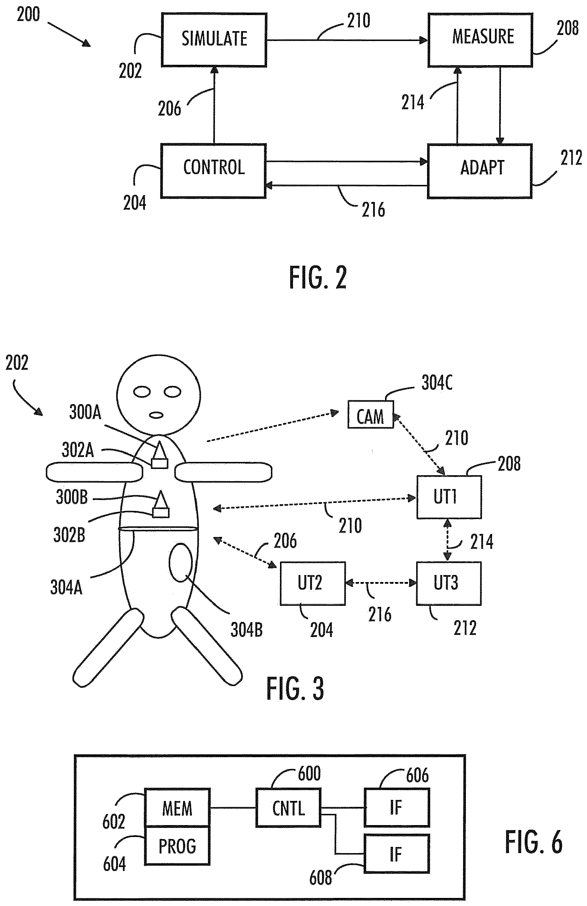

Fig.2 illustrates a simplified example of an apparatus of an embodiment. The apparatus 200 comprises simulating means or simulator 202 for obtaining simulated physiological response of a body of a living being to vital functions of the body. The apparatus further comprises controlling means or device 204 for controlling 206 the simulating means, measuring means or device 208 for measuring 210 the simulated physiological response to obtain measurement data and adapting means or adapting device 212 for comparing the measurement data to predetermined data and adapting 214, 216 the measuring and controlling means based on the comparison.

In an embodiment, with the arrangement or Fig.2 it is possible to perform simulation of vital functions such as respiration and cardiac functions as well as perform multi-sensor monitoring of vital signs caused by the vital functions. In an embodiment, the simulator may be utilised for tuning and/or training of the measurement and analysis system that determines the state of health or mind of the monitored living being. Given that an accurate simulator of the physical/electrical response is available, the simulator can be used to generate unlimited amounts of training data for automatic model and algorithm tuning and training. This enables the possibility of a feed-back loop where the analysis algorithm performance is improved by using the simulator data and the operation of the simulator can be improved by using the analysis results.

In an embodiment, with the tuned and trained measurement system, it is possible to jointly use respiration and cardiac function measurements to determine the higher level state of a living being. As respiration patterns only tell about part of the living being state, including the cardiac function into the analysis will lead to more accurate analysis. Furthermore, the joint use of image-based (e.g., thermal or normal camera) and movement-based (inertial measurement using or breathing belt) sensors also allow for identification of conditions that would be hard to detect based on one of them only.

The proposed simulation arrangement enables training and tuning the measurement system in such a manner that the above mentioned advanced results maybe obtained.

In an embodiment, the simulating means 202 are realised with a human shape torso or robot having a set of elements; a set of actuators producing movement of the set of elements to simulate movements of a human body caused by the vital functions of the human body and a set of sensors producing sensor output based on the movements.

In an embodiment, the simulating means 202 are realised with at least one processor and at least one memory including computer program code; wherein the at least one memory and the computer program code configured to, with the at least one processor, cause the means at least to perform: obtaining, utilising a physical model, simulated movement trajectories simulating movements of a body caused by the vital functions of the body and determine sensor output based on the movements.

Fig.3 illustrates a simplified schematic example of an embodiment where the simulating means or device 202 are realised with a physical human shape torso or robot, which is configured to mimic different vital functions such as respiration patterns or cardiac functions in different medical or physical conditions, or states of mind. Corresponding simulating means or device 202 may also have the shape of a living being other than human. The simulating means (or simulator 202 for short) may be a mechanical device with a realistic skin than has a set of elements 300A, 300B and a set of actuators or motors 302A, 302B producing movement of the set of elements 300A, 300B to simulate movements of a human body caused by the vital functions of the human body. The movements may be produced by moving or vibrating the element or a part of an element. The set of elements and actuators may be configured to control the physical movement of the artificial skin, torso, eyes, mouth, and other body parts. In an embodiment, there may also be actuators for pulsation, sound, temperature, and other features that can be used to mimic human behavior in different medical conditions.

The actuator or motors 302A, 302B may be controlled by controlling means or controlling device 204. The controlling means or device may be a separate computing device outside the simulating means, as illustrated in Fig. 3, or they may be realised with an embedded processor attached to the actuators or motors. The controlling means may be configured to produce predefined, programmable, or remotely controlled movement patterns of the set of elements. The controlling means or controllers 204 may be, for example, proportional-integral-derivative (PID) controllers, bang-bang-controllers, optimal controllers such as linear quadratic (LQ) controllers, or model predictive controllers (MPC). These controllers may be given movement trajectories and the controllers may be programmed to convert these trajectories into voltage signals that drive the motors and other actuators to generate the desired movement of the set of elements. In an embodiment, these trajectories may be optimized using feedback from sensor measurements as described later.

In an embodiment, the simulator 202 is measured with a set of sensors 304A, 304B, 304C. the sensors may be in embedded in the simulator, attached to the simulator or outside the simulator. The set of sensors may measure the movement and/or sound produced by the simulator. Examples of possible sensors include an accelerometer, a gyroscope, a magnetometer, a microphone, a thermometer, a thermal camera, a camera, a radar, a breathing belt, an inductance sensor, an electrocardiography sensor, a signal phase sensor, a received signal strength sensor, a pulse-oximeter sensor, capacitive sensor and light detection and ranging (LI-DAR) sensor. Further examples comprise wearable devices (such as smart bracelets, smartwatches, smart rings, etc.), thermal sensors, capacitive or inductive sensors, pressure sensors, strain gauges and contact-free measurement techniques based on ambient signals. The sensors may also be employed in custom, application-specific sensor systems. In an embodiment, breathing/cardiac sounds may be monitored and speech distress generated by the simulator may be collected and analysed. In the example of Fig.3, there are two sensors 304A, 304B, in the simulator and one 304C outside the simulator. The sensor 304C may be, for example, a thermal camera or a normal camera possibly including microphone, radar, or radio transmitter/receiver which remotely measures the simulator 202.

In an embodiment, the simulated vital signs, such as respiratory and cardiac signals, can be measured by one or a combination (fusion) of different hardware and software sensors.

In an embodiment, the simulated physiological response data generated by the set of sensors is gathered at measuring means 208 to obtain and generate measurement data. In an embodiment, the data generated by the sensors may be collected by an embedded processor in the simulator and transmitted over a communications channel 210 to the measuring means 208 or the data from the sensors may be directly sent over the communications channel 210. The measuring means 208 may also be configured to send commands to the sensors or embedded processors.

The communications channel 210 may be, for example, a wireless communication channel such as Bluetooth® or Wireless Local Area Network (WLAN, WiFi) based on IEEE 802.11 standard, or worldwide interoperability for microwave access (WiMAX®), or other wired or wireless communication method.

In an embodiment, the measuring means 208 may be a computing device such as a personal computer, a smartphone, a tablet, or any processing device comprising required interfaces.

In an embodiment, there are adapting means 212 configured to obtain measurement data from the measuring means 208 and compare the measurement data to predetermined data and adapt 214, 216 the measuring and controlling means 204, 208 based on the comparison. In an embodiment, the adapting means 212 may be a computing device such as a personal computer, a smartphone, a tablet, or any processing device comprising required interfaces. The adapting means 212 may be configured to process the measurement data for improving or modifying the control patterns generated by the controller 204 of the simulator.

In an embodiment, the measuring means 208 and adapting means 212 may be realised with a single computing device. In an embodiment, also the controlling means 204 may be realised with the same device in whole or in part. The controlling means may also be partly embedded in the simulator 202 and partly realised with an external device.

In an embodiment, an initial implementation of the control system in the simulator 202 can be as follows. First a medical person and control engineer together program breathing and cardiac patterns to the controlling device 204 so that that the set of elements 300A, 300B of the simulator 202 produce movements corresponding to different breathing and cardiac patterns that mimic reality based on medical doctor's judgement. The simulator can also be used to generate e.g. speech which corresponds to a certain distress level.

The generation of these patterns can be, for example, done by a computer program that runs in the embedded processor or external computer or smartphone such that when it is given a command to produce e.g. normal expirium or inspirium, alveolar breathing pattern, obstruction of the bronchus or bronchioles, paradoxical breathing expirium phase, or paradoxical breathing inspirium phase, it produces it using predefined breathing rate and strength and cardiac response.

The aforementioned patterns can also be generated with help of a person who mimics the patterns and they are then programmed accordingly to the simulator.

In an embodiment, the controlling means comprise a physical model configured to calculate control parameters for the simulating means to generate movements of a human body caused by the vital functions of the human body, the physical model comprising adaptable parameters.

After the initial control algorithms, the patterns can be run while monitoring the simulator with the various sensors. The measurement means or device 208 receives and processes the sensor readings.

In an embodiment, the sensor readings may first be run through a Kalman filter, particle filter, or similar algorithm that uses sensor fusion to combine the movement sensor readings into physical movement, reduces the noises in the signals, and possibly combines the thermal/normal camera, microphones, and other sensor readings to estimate the underlying physical movement and sound signals.

As many of the commanded movements are known, it is also possible to use system identification methods based on e.g. autoregressive (AR) models, finite impulse response (FIR) models, neural networks, or Gaussian processes to form a data-driven algorithm that is able to convert processed or raw sensor data into physical movement parameters or sounds.

In an embodiment, from sensor data signals it is also possible to extract features such as derivatives, frequencies, signal shapes, and other parameters and feed them to classifier such as support vector machine (SVM), random forest (RF), Gaussian process classifier, or similar to determine the breathing or cardiac pattern that the simulator was commanded to perform.

In an embodiment, it is possible to collect data from the simulator and use deep learning methods to automatically generate the features from processed or raw sensor data to reproduce the physical movement or sound, or to directly classify the breathing and cardiac patterns.

Thus, in an embodiment, the measurement means or device 208 receives sensor data and based on the data obtains measurement data. In an embodiment, the measurement data comprises the vital signs performed by the simulator 202.

In an embodiment, the measuring means comprise a physical model configured to calculate, based on the sensor outputs, movements of a human body caused by the vital functions of the human body, the physical model comprising adaptable parameters.

The adapting means or adapting device 212 is configured to receive the measurement data or the interpreted vital signs. The adapting means or adapting device 212 is further configured to compare the received data to predetermined data and adapt and control the measuring and controlling means or devices 204, 208 based on the comparison.

Thus, in an embodiment, the adapting means or adapting device 212 compare the vital signs determined based on the data received from the sensors to the vital signs that the simulator was controller to generate. The adapting means or adapting device 212 may then adapt the operation of either or both the measuring and controlling means or devices 204, 208 so that the vital signs generated by the simulator and/or the determination made based on the sensor data better producers correct results.

In an embodiment, the adapting means or adapting device 212 may also be used to train and tune the measuring and controlling means or devices 204, 208 as follows.

The control patterns performed by the control 204 may be artificially varied such that the simulator 202 is controlled to perform patterns that are similar to programmed patterns, but differ in various ways. This varying can be done, for example, by randomly or deterministically varying the control trajectories while still validating by a medical person that the patterns are physically feasible.

The modified patterns can be used to re-teach the measuring means or device 208 so that it is able to generate the movement and classify the breathing and cardiac patterns despite the variations. The sensor measurements can be further validated either by a medical person or by automatic testing to make sure that the patterns are still within the medically feasible limits.

In an embodiment, different movements of the simulator may also be used to simulate realistic external movements to make the measurement system robust to them. It is possible even to place the simulator to a realistic environment (such as sports equipment, car) and perform measurements in that environment to improve the data processing. The aim of this is to generate data from vital signs while movement which allows for the system become robust to these movements.

In an embodiment, the sensor data may also be used as feedback to the controllers in order to produce more smooth or otherwise more physically realistic signals for simulation. The relationship with given control commands and measured movements can be further used to tune the measurement system as well as the control model and commands in the control devices.

In an embodiment, the computations performed by the adapting means or adapting device 212 may be implemented, for example, by using a deep or shallow neural network, recursive neural network, tree or forest classifier, Bayesian classifier, Kalman filter, particle filter, Gaussian process regressor or classifier, Gaussian process state-space model, or a system identification method that combines these parts. These can be further combined with statistical or non-statistical parameter estimation method.

The aforementioned steps can be performed multiple times in order to use the sensor feedback to enhance the simulation.

The control and feedback functions of the simulating arrangement can be implemented, for example, using Bellman's dynamic programming, PID controller, LQ-controller, reinforcement learning, or an adaptive control method that combines these with the aforementioned learning, adaptive filtering, and system identification components. The sounds can be generated with a sound or speech synthe sizer or by using prerecorded sounds.

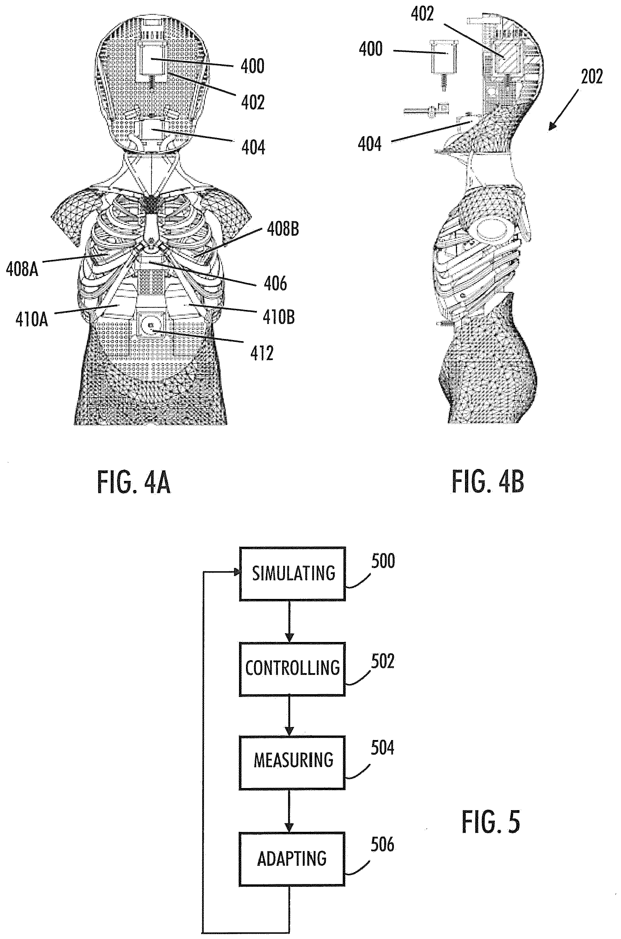

Figs. 4A and 4B illustrate an example of an embodiment where the simulating means or device 202 are realised with a physical human shape torso or robot. The simulating means or simulator 202 for short may be a mechanical device with a realistic skin than has a set of elements 300A, 300B and a set of actuators or motors 302A, 302B producing movement of the set of elements 300A, 300B to simulate movements of a human body caused by the vital functions of the human body. The movements may be produced by moving or vibrating the element or a part of an element.

The simulating means or device 202 of Figs. 4A and 4B can mimic respiratory movement in different parts of the face and body. In an embodiment, the movements are technically conducted by actuators stepper motors connected to the flexible compartments of the torso and nasal area of the face. The mode of the movement (i.e. respiratory pattern) can be modified by using different input data in the steering system of the simulator.

In the example of Figs. 4A and 4B, the torso or robot 202 comprises actuators or motors for nasal 400, sternocleidomastoideus 402, neck 404, sternum or breastbone 406, thorax upper right 408A, thorax upper left 408B, diaphragm 412, right rib 410A and left rib 410B.

As mentioned, in an embodiment the simulating means or device 202 may be realised as a computer program code executed by at least one processor and at least one memory including the computer program code. The executed computer program code may utilise a physical model and calculate movement trajectories simulating movements of a human body caused by the vital functions of the human body; and determine virtual sensor output based on the calculated movements.

Thus, a software-based simulator may use a physical model to mimic the physical and electrical respiration and cardiac function characteristics related to the medical condition or state of mind. Computationally generated virtual sensors may then be attached to the software-model such that they produce realistic measurements of the implied respiration and cardiac signals. The computations for analysing this data and for tuning the software-simulator control can be implemented using similar methods as in the case of a physical simulator.

The simulator 202 can also be implemented as a hybrid system that contains both physical and software-based simulations. For example, we can use a physical simulator for simulating the stationary being's respiration and cardiac patterns while the macroscopic movement can be simulated using computational means. The macroscopic movement can also be generated with another physical simulator and the two simulators are computationally combined. Alternatively, we can, for example, physically simulate only the macroscopic movement and use software to simulate the respiration and cardiac patterns on top of that.

Fig. 5 is a flowchart illustrating an embodiment.

In step 500, physiological response of a human body to vital functions of the human body is simulated.

In step 502, the simulation is controlled.

In step 504, the simulated physiological response is measured and analysed to obtain measurement data. In an embodiment, the measurement data comprises estimation of the physiological response of the human body.

In step 506, the measurement data is compared to predetermined data and the measuring and controlling is adapted based on the comparison. In an embodiment, the estimated physiological response is compared to the physiological response the simulation was controlled to generate.

Figure 6 illustrates a simplified example of a computing device. In an embodiment, the computing device may be the measurement device 208, the controlling device 204 or the adapted device 212, for example.

The device of the example includes one or more control circuitries or processing circuits (CNTL) 600 configured to control at least part of the operation of the device.

The device may comprise one or more memories (MEM) 602 for storing data. Furthermore, the memory may store software (PROG) 604 executable by the control circuitry 600. The memory may be integrated in the control circuitry.

The device may comprise one or more interface (IF) 606, 608. The interfaces are operationally connected to the control circuitry 600. One or more of the interfaces may be a wired and/or wireless transceiver enabling the device to communicate with other devices. The device may also comprise an antenna arrangement (not shown).

The software 604 may comprise a computer program comprising program code means adapted to cause the control circuitry 600 of the device to control the interfaces. The software 604 may comprise a computer program comprising program code means adapted to cause the control circuitry 600 of the device to execute all or some of the functions described above.

One of the interfaces may also be user interface operationally connected to the control circuitry 600. The user interface may comprise a display which may be touch sensitive, a keyboard or keypad, a microphone and a speaker, for example.

The use of multiple sensors together with a simulator of the physical and/or electrical phenomena enables more robust and accurate analysis of vital functions such as the respiration and cardiac functions more accurately than before. For example, the physical simulator allows for mimicking of disturbances generated by movements and sensor noises more accurately than could be achieved with pure first-principles modeling.

In an embodiment, the model used for the control algorithm in control means or device 204 may comprise a dynamic model dx(t)/dt = A x(t) + B u(t) and an output model y(t) = C x(t) + error, where x(t) is the vector-valued state, y(t) is the physical movement, and A, B, C are adaptable matrices, and u(t) is the control signal. Given the matrices A, B, and C, the control problem can be solved, for example, by using an LQ controller which minimizes a quadratic error functional of the state and control. The measuring means or device 208 may be used to estimate the realized output y(t) and the realized output can be compared in the adapting means or adapting device 212 with a desired output. The result of this comparison can be used to tune the control algorithm by adjusting the control profiles as well as by adapting the parameters matrices A, B, and C. A discrete time version of the above model could also be used, and the LQ controller could be replaced with any other form of controller such as PID.

In an embodiment, the measuring means or device system 208 may utilise a measurement model of the form z(t) = H x(t) + error, where H is an adaptable matrix. Given the dynamic model and matrix H, the measurement problem can be solved e.g. using a Kalman filter. Given one of the signals x(t), u(t), y(t), or estimates of them, it is possible to adapt the matrix H so that the Kalman filter can be used to better recover the signal x(t). The Kalman filter can also be implemented in discrete time, the model could be non-linear, and the estimator could be replaced e.g. with a particle filter or more general Bayesian filter.

In an example embodiment, the adapting means or adapting device 212 receives the control signal u(t) from the controlling means or device 204, and realized output estimate y(t) from the measuring means or device 208. By comparing these signals, the adapting means or device 212 may be configured to compute new parameters A, B, and C, and send them to the controlling means or device 204.

In an embodiment, the adapting means or device 212 receives the commanded state trajectory x(t) from the controlling means or device 204 and sensor output z(t) from measurement means or device 208. Based on these, the adapting means or device 212 may be configured to compute a new matrix H and send it to the measuring means or device 208.

In relation to other machine learning and adaptive filtering approaches the present system needs much less data, if any, collected from living beings. As the simulator can be run extensive amounts of time, it may provide a continuous stream of measurement data which would be impossible to generate from living subjects. The large amount of data also ensures the robustness of the analysis, as various different conditions can be included in the dataset. The adaptation ability for differing conditions can be enabled simply by programming additional patterns to the simulation which are then used to generate training data and for training the system.

Furthermore, the simulator can also be put into a realistic environment (e.g. hospital, car, bed) to generate realistic data for the method training. This way the natural background phenomena occurring in the environment can be automatically incorporated in the analysis system.

It will be obvious to a person skilled in the art that, as the technology advances, the inventive concept can be implemented in various ways. The invention and its embodiments are not limited to the examples described above but may vary within the scope of the claims.

## Claims

1. An apparatus comprising:
simulating means for obtaining simulated physiological response of a living body to vital functions of the living body;
controlling means for controlling the simulating means;
measuring means for measuring the simulated physiological response to obtain measurement data; and
adapting means for comparing the measurement data to predetermined data and adapting the measuring and controlling means based on the comparison.

2. The apparatus according to claim 1, the simulating means comprising:
a torso having a shape of a living being or robot having a set of elements;
a set of actuators producing movement of the set of elements to simulate movements of a living being caused by the vital functions of the body of a living being;
a set of sensors producing sensor output based on the movements.

3. The apparatus according to claim 2, the controlling means comprising:
one or more controllers generating control data for controlling the set of actuators to perform movement trajectories simulating movements of the body of a living being caused by the vital functions of the body;
receive adapting data from the adapting means;
adjust the control data based on the adapting data.

4. The apparatus according to any preceding claim 2 to 3, the set of sensors comprising in the apparatus, attached to the apparatus or outside the apparatus one or more of the following:
an accelerometer, a gyroscope, a magnetometer, a microphone, a thermometer, a thermal camera, a camera, a radar, a breathing belt, an inductance sensor, an electrocardiography sensor, a signal phase sensor, a received signal strength sensor, capacitive sensor, pulse-oximeter sensor, light detection and ranging sensor.

5. The apparatus according to claim 1, the simulating means comprising:
at least one processor and at least one memory including computer program code; wherein the at least one memory and the computer program code configured to, with the at least one processor, cause the means at least to perform:
obtaining, utilising a physical model, simulated movement trajectories simulating movements of a body of a living being caused by the vital functions of the body; and
determine sensor output based on the movements.

6. The apparatus according to claim 2 or 5, the measuring means comprising:
a physical model configured to calculate, based on the sensor outputs, movements of the body of a living being caused by the vital functions of the body, the physical model comprising adaptable parameters.

7. The apparatus according to any preceding claim, the controlling means comprising:
a physical model configured to calculate control parameters for the simulating means to generate movements of the body of a living being caused by the vital functions of the body, the physical model comprising adaptable parameters.

8. The apparatus according to claim 6 and 7, the adapting means being configured to
receive and combine the physical models or adaptable parameters of the controlling and measuring means,
receive information on the measurement data from the measuring means;
receive information on the control data from the controlling means; and
adapts measurement means and controlling means based on the received information.

9. The apparatus according to any preceding claim, the controlling means comprising user interface for receiving control data.

10. A method comprising:
simulating physiological response of a body of a living being to vital functions of the body;
controlling the simulation;
measuring the simulated physiological response to obtain measurement data;
comparing the measurement data to predetermined data and adapting the measuring and controlling based on the comparison.

11. The method according to claim 10, further comprising:
simulating the physiological response of the body of a living being utilising a torso having a shape of a living being or robot having a set of elements;
producing movement of the set of elements utilising a set of actuators to simulate movements of the body caused by the vital functions of the body;
producing based on the movements sensor output utilising a set of sensors.

12. The method according to claim 11, further comprising:
generating control data for controlling the set of actuators to perform movement trajectories simulating movements of the body of a living being caused by the vital functions of the body;
receiving adapting data;
adjusting the control data based on the adapting data.

13. The method according to claim 10, further comprising:
obtaining, utilising a physical model, simulated movement trajectories simulating movements of a body of a living being caused by the vital functions of the body; and
determine sensor output based on the movements.

14. The method according to any preceding claim 10 to 13, further comprising:
configured to calculating, by a physical model, based on the sensor outputs, movements of the body of a living being caused by the vital functions of the body, the physical model comprising adaptable parameters.

15. The method according to any preceding claim 10 to 14, further comprising:
configured to calculating, by a physical model, control parameters for generating movements of the body of a living being caused by the vital functions of the body, the physical model comprising adaptable parameters.
